# EUROPEAN PATENT SPECIFICATION

(11) **EP 3 062 760 B1**
(45) Date of publication and mention of the grant of the patent: **16.08.2017**
(21) Application number: 14787065.3
(22) Date of filing: 06.10.2014
(51) Int. Cl.: A61K 8/26, A61Q 5/08, A61Q 5/10, A61K 8/31

(54) **SECOND AGENT COMPOSITION FOR HAIR DYEING OR BLEACHING**
ZWEITE KOMPONENTE EINER ZUSAMMENSETZUNG ZUR FÄRBUNG ODER BLEICHUNG VON HAAREN
DEUXIEME AGENT D'UNE COMPOSITION POUR LA COLORATION OU LA DECOLORATION DES CHEVEUX

(30) Priority: 30.10.2013 JP 2013225703
(43) Date of publication of application: 07.09.2016
(73) Proprietor: Henkel AG & Co. KGaA, 40589 Düsseldorf (DE)
(72) Inventor: MATSUTANI, Akira, Tokyo 140-0002 (JP); KAWAI, Masaki, Tokyo 140-0002 (JP); MACHIDA, Shoji, Tokyo 140-0002 (JP); NAGAFUCHI, Shoko, Tokyo 140-0002 (JP)
(86) International application number: PCT/JP2014/077121
(87) International publication number: WO 2015/064336

(56) References cited:
- EP-A1- 1 584 323
- WO-A1-2011/076792

## Description

### [Technical Field]

The present invention relates to a second agent composition for hair dyeing or bleaching which is used by being mixed with a first agent composition containing an alkali agent and which is excellent in firmness/resilience and manage ability of hair after a dyeing or bleaching treatment.

### [Background Art]

A process in which a first agent composition containing an alkali agent is mixed with a second agent composition containing hydrogen peroxide immediately before use, and then, the resulting hair cosmetic is applied to hair to dye or bleach hair is known as a process for hair dyeing or bleaching. In recent years, hair dyeing or bleaching became common and a higher dyeing or bleaching power is demanded.

On the other hand, hair is damaged due to chemical influences caused by hair dyeing or bleaching, as a result, firmness/resilience of hair is sometimes reduced and manage ability of hair is sometimes lost after a dyeing or bleaching treatment.

A composition containing a cationic polymer is studied so as to obtain manage ability of hair after a dyeing or bleaching treatment (Patent Documents 1 and 2). However, the composition has to contain a large amount of the cationic polymer in order to obtain good manage ability. In addition, a large quantity of the cationic polymer sometimes hardens hair to cause a creaking of hair depending on the type of hair.

In order to increase a dyeing or bleaching power, an attempt to sufficiently accelerate the effect of an oxidizing agent by adding an oil to a hair cosmetic was made (Patent Documents 3 to 7). However, the previous hair cosmetics do not achieve sufficient firmness/resilience and manage ability of hair after a dyeing or bleaching treatment.

### [Citation List]

### [Patent Literature]

Patent Document 1: JP 2005-23023 A
Patent Document 2: JP 2005-179222 A
Patent Document 3: JP 2007-217291 A
Patent Document 4: JP 2007-217292 A
Patent Document 5: JP 2007-217293 A
Patent Document 6: JP 2003-81792 A
Patent Document 7: JP 2003-55174 A
WO2011/07692 discloses oxidative dying compositions comprising non-ionic and cationic surfactants, oxidation agent and liquid oils.

### [Summary of Invention]

### [Technical Problem]

Thus, an object of the present invention is to provide a second agent composition for hair dyeing or bleaching which enables sufficient dyeing or bleaching of hair and which is excellent in firmness/resilience and manage ability of hair after a dyeing or bleaching treatment. Furthermore, other object of the present invention is to provide a second agent composition, wherein the second agent composition is excellent in storage stability, a dyeing or bleaching agent composition has good operability, hair after a dyeing or bleaching treatment is excellent in feeling, and uniform dyeing or bleaching of hair can be achieved.

### [Solution to Problem]

The present inventors have intensively made researches. As a result, they have found that the problems are solved by a second agent composition according to the present invention which contains particular components in a specific weight ratio and which stably contains an oil with a small amount of surfactants.

That is, the problems are solved by the following specific embodiments.
(1) A second agent composition for hair dyeing or bleaching which is used by being mixed with a first agent composition containing an alkali agent, wherein the second agent contains following components (A) to (C):
   (A) (a1) from 10 to 30 % by weight of at least one oil which is in a liquid form at 25°C and (a2) at least one oil which is in a solid form at 25°C, wherein, the oil which is in a solid form at 25°C comprises a higher alcohol which is in a solid form at 25°C, and the weight ratio (A)/(a2) of the total weight (A) of (a1) and (a2) to (a2) is from 3 to 8,
   (B) (b1) at least one cationic surfactant and (b2) at least one nonionic surfactant, wherein, the cationic surfactant comprises an alkyltrimethylammonium chloride which has from 16 to 22 carbon atoms in the alkyl group thereof, and the total weight (B) of (b1) and (b2) is from 0.5 to 1.8 % by weight, and
   (C) from 0.1 to 12 % by weight of an oxidizing agent, wherein, the weight ratio (A)/(B) of (A) to (B) is from 5 to 30.
(2) The composition according to (1), wherein the weight ratio (b1)/(b2) of (b1) to (b2) is from 0.1 to 1.
(3) The composition according to (1) or (2), wherein the higher alcohol comprises at least one selected from the group consisting of cetyl alcohol, stearyl alcohol and cetostearyl alcohol.
(4) The composition according to any one of (1) to (3), wherein the nonionic surfactant comprises at least one polyoxyethylene alkyl ether.

### [Advantageous Effects of Invention]

According to the present invention, it is possible to impart good firmness/resilience and good manageability to hair after a dyeing or bleaching treatment in addition to a sufficient hair dyeing or bleaching result. In addition, the second agent composition according to the present invention has a good emulsified state and good storage stability, a dyeing or bleaching agent composition has good operability when the dyeing or bleaching agent composition is applied, hair after a dyeing or bleaching treatment has a good feeling, and uniform dyeing or bleaching of hair is achieved.

### [Description of Embodiments]

The composition according to the present invention is a second agent composition for hair dyeing or bleaching. The composition according to the present invention can dye or bleach hair by being mixed with a first agent composition containing an alkali agent and optionally with other composition to obtain a dyeing or bleaching agent composition and applying the resulting dyeing or bleaching agent composition to hair.

The composition according to the present invention contains (A) (a1) from 10 to 30 % by weight of at least one oil which is in a liquid form at normal temperature and (a2) at least one oil which is in a solid form at normal temperature so as to achieve good storage stability of the second agent composition, good operability of the dyeing or bleaching agent composition, a sufficient hair dyeing or hair bleaching result and good firmness/resilience and good manage ability of hair after a dyeing or bleaching treatment. The term "normal temperature" means 25°C in the present invention.

### (a1) Oil which is in a liquid form at normal temperature

The oil which is in a liquid form at normal temperature is not particularly limited. The examples of the oil which is in a liquid form at normal temperature include, for example, an ester oil, a hydrocarbon oil, a silicone oil, an animal oil, a vegetable oil, a higher alcohol which is in a liquid form at normal temperature and a higher fatty acid which is in a liquid form at normal temperature. The examples of the ester oil include, for example, an esterification product of a fatty acid having from 6 to 24 carbon atoms such as caproic acid, caprylic acid, 2-ethylhexanoic acid, capric acid, lauric acid, isotridecanoic acid, myristic acid, palmitic acid, palmitoleic acid , stearic acid, isostearic acid, oleic acid, elaidic acid, petroselinic acid, linolic acid, linoleic acid, eleostearic acid, arachic acid, gadoleic acid, behenic acid and erucic acid with an alcohol such as isopropyl alcohol, caproic alcohol, capryl alcohol, 2-ethyl hexyl alcohol, capric alcohol, lauryl alcohol, isotridecyl alcohol, myristyl alcohol, cetyl alcohol, palmitoleyl alcohol, stearyl alcohol, isostearyl alcohol, oleyl alcohol, elaidyl alcohol, petroselinyl alcohol, linolyl alcohol, linolenyl alcohol, eleostearyl alcohol, arachyl alcohol, gadoleyl alcohol, behenyl alcohol, erucyl alcohol and brassidyl alcohol. The examples of the ester oil further include a dicarboxylic acid ester such as di-n-butyl adipate, di-(2-ethylhexyl) adipate, di-(2-ethylhexyl) succinate and diisotridecyl acelate; diolester, such as ethylene glycol dioleate, ethylene glycol diisotridecanoate, propylene glycol di-(2-ethyl hexanoate), propylene glycol diisostearate, propylene glycol dipelargonate, butanediol diisostearate, and neopentyl glycol dicaprylate; and a complex ester such as diacetyl glycerol monostearate. The examples of the hydrocarbon oil include, for example, liquid paraffin, dioctyl cyclohexane, α-olefin oligomer, light isoparaffin, light liquid isoparaffin, squalane, polybutene and liquid isoparaffin. The examples of the silicone oil include, for example, dimethyl polysiloxane, methyl phenyl polysiloxane, cyclic silicone, and amino-modified, fatty acid-modified, alcohol-modified, polyether-modified, epoxy-modified, fluorine-modified silicone compound and alkyl-modified silicone compound. The examples of the animal oil and the vegetable oil include, for example, macadamia nut oil, coconut oil, amaranth seed oil, peach kernel oil, avocado oil, olive oil, rapeseed oil, sesame oil, jojoba oil, soybean oil, peanuts oil, Oenothera biennis oil and Camellia sinensis oil. The examples of the higher alcohol which is in a liquid form at normal temperature include, for example, hexyl decanol, isostearyl alcohol, octyl dodecanol, decyl tetradecanol, oleyl alcohol etc. The examples of the higher fatty acid which is in a liquid form at normal temperature include, for example, isostearic acid, oleic acid and linolic acid etc. Each of these oils which is in a liquid form at normal temperature can be used in the composition according to the present invention alone, or two or more kinds of them can be used in combination.

The composition according to the present invention preferably contains a hydrocarbon oil and/or an ester oil and more preferably contains an ester oil, from the view point of the emulsified state of the composition and manage ability of hair.

The amount of the oil which is in a liquid form at normal temperature is 10 % by weight or more based on the total weight of the second agent composition so as to obtain a sufficient hair dyeing or hair bleaching effect, good operability of the dyeing or bleaching agent composition, good firmness/resilience and manage ability of hair after a dyeing or bleaching treatment. The amount of the oil which is in a liquid form at normal temperature is preferably 15 % by weight or more based on the total weight of the second agent composition from the view point of dyeing or bleaching power and firmness/resilience and manage ability of hair after a dyeing or bleaching treatment. The amount of the oil which is in a liquid form at normal temperature is 30 % by weight or lower based on the total weight of the second agent composition so as to obtain a good emulsified state and good storage stability of the composition according to the present invention, and good operability of the dyeing or bleaching agent composition. The amount of the oil which is in a liquid form at normal temperature is preferably 25 % by weight or lower and more preferably 20 % by weight or lower based on the total weight of the second agent composition from the view point of storage stability of the composition according to the present invention.

### (a2) Oil which is in a solid form at normal temperature

The oil which is in a solid form at normal temperature is not particularly limited. The examples of the oil which is in a solid form at normal temperature include, for. example, a hydrocarbon, an animal fat, waxes, a higher fatty acid, esters, which are in a solid form at normal temperature. In the present invention, the phrase "in a solid form at normal temperature" means that a substance is in a solid or paste form at a temperature of 25°C. For example, a hydrocarbon such as paraffin wax and waxes such as bees wax.

In the composition according to the present invention, the oil which is in a solid form at normal temperature comprises a higher alcohol which is in a solid form at normal temperature. The higher alcohol generally means a monohydric alcohol having a carbon number of 6 or more. In the present invention, examples of the higher alcohol which is in a solid form at normal temperature include, for example, a higher alcohol having a carbon number of 12 or more, specifically, myristyl alcohol, cetyl alcohol, cetostearyl alcohol (a mixture of cetyl alcohol and stearyl alcohol) , stearyl alcohol, behenyl alcohol, arachyl alcohol, lanolin alcohol. Each of these higher alcohols which is in a solid form at normal temperature can be used in the composition according to the present invention alone, or two or more kinds of them can be used in combination. In addition, at least one higher alcohol which is in a solid form at normal temperature can be used in combination with at least one oil which is in a solid form at normal temperature described in the preceding paragraph.

The composition according to the present invention preferably contains a higher alcohol having a carbon number of 16 or more and more preferably contains, for example, cetyl alcohol, stearyl alcohol or cetostearyl alcohol from the view point of a emulsified state and storage stability of the composition and manage ability of hair after a dyeing or bleaching treatment.

The amount of the higher alcohol which is in a solid form at normal temperature is preferably 2 % by weight or more, more preferably 3 % by weight or more, even more preferably 4 % by weight or more based on the total weight of the second agent composition from the view point of emulsified state and storage stability of the composition according to the present invention and manage ability of hair after a dyeing or bleaching treatment. The amount of the higher alcohol which is in a solid form at normal temperature is preferably 12 % by weight or lower, more preferably 10 % by weight or lower, even more preferably 8 % by weight or lower based on the total weight of the second agent composition from the view point of storage stability of the composition according to the present invention and operability of the dyeing or bleaching agent composition.

The amount of the higher alcohol which is in a solid form at normal temperature is preferably 50 % by weight or more, more preferably 70 % by weight or more, even more preferably 80 % by weight or more, particularly preferably 90 % by weight or more and very particularly preferably 100 % by weight based on the total weight of oil which is in a solid form at normal temperature from the view point of emulsified state and storage stability of the composition according to the present invention and operability of the dyeing or bleaching agent composition.

In the composition according to the present invention, the weight ratio (A)/(a2) of the total weight (A) of (a1) an oil which is in a liquid form at normal temperature and (a2) an oil which is in a solid form at normal temperature to (a2) oil which is in a solid form at normal temperature is from 3 to 8, preferably from 4 to 7 and more preferably from 4 to 5 so as to obtain good emulsified state of the composition according to the present invention, good operability of the dyeing or bleaching agent composition and good manage ability of hair after a dyeing or bleaching treatment.

The composition according to the present invention contains (B) (b1) at least one cationic surfactant and (b2) at least one nonionic surfactant so as to obtain good storage stability of the composition, good operability of the dyeing or bleaching agent composition and smooth feeling of hair after a dyeing or bleaching treatment.

### (b1) Cationic surfactant

The examples of the cationic surfactant include, for example, ethyl sulfate lanolin fatty acid amino propyl ethyl dimethyl ammonium, behenyl trimethyl ammonium chloride, octadecyl ammonium chloride, octyl dihydroxyethyl methyl ammonium chloride, dialkyl (12-15) dimethyl ammonium chloride, dialkyl(14-18) dimethyl ammonium chloride, dicocoyl dimethyl ammonium chloride, distearyl dimethyl ammonium chloride, dicetyl dimethyl ammonium chloride, di(polyoxyethylene)oleyl methyl ammonium chloride, stearyl dihydroxyethyl betain sodium chloride, stearyl dimethyl benzyl ammonium chloride, stearyl trimethyl ammonium chloride, cetyl trimethyl ammonium chloride, cetyl pyridinium chloride, benzalkonium chloride, benzethonium chloride, polyoxyethylene (1) polyoxypropylene (25) diethyl methyl ammonium chloride, myristyl dimethyl benzyl ammonium chloride, methyl benzethonium chloride, lauryl trimethyl ammonium chloride, lauryl pyridinium chloride, alkyl isoquinolinium bromide, stearyl trimethyl ammonium bromide, cetyl trimethyl ammonium bromide, lauryl trimethyl ammonium bromide, stearyl trimethyl ammonium saccharin and cetyl trimethyl ammonium saccharin.

In the composition according to the present invention, the cationic surfactant comprises alkyltrimethylammonium chloride which has from 16 to 22 carbon atoms in the alkyl group thereof. The examples of alkyltrimethylammonium chloride having from 16 to 22 carbon atoms in the alkyl group thereof include, for example, cetyl trimethyl ammonium chloride, stearyl trimethyl ammonium chloride and behenyl trimethyl ammonium chloride. Each of these cationic surfactants can be used in the composition according to the present invention alone, or two or more kinds of them can be used in combination. In addition, at least one of these cationic surfactant can be used in combination with at least one cationic surfactant described in the preceding paragraph. The composition according to the present invention preferably contains cetyltrimethylammonium chloride and/or stearyl trimethylammonium chloride from the view point of storage stability of the composition, operability of the dyeing or bleaching agent composition and smooth feeling of hair after a dyeing or bleaching treatment.

The amount of the alkyltrimethylammonium chloride having from 16 to 22 carbon atoms in the alkyl group thereof is preferably 50 % by weight or more, more preferably 70 % by weight or more, even more preferably 80 % by weight or more, particularly preferably 90 % by weight or more and very particularly preferably 100 % by weight based on the total amount of the cationic surfactant from the view point of storage stability of the composition, operability of the dyeing or bleaching agent composition and smooth feeling of hair after a dyeing or bleaching treatment.

### (b2) Nonionic surfactant

The nonionic surfactant is not particularly limited. The examples of the nonionic surfactant include a polyoxyethylene type nonionic surfactant such as polyoxyethylene alkyl ether and polyoxyethylene phenyl ether, ester type nonionic surfactant such as polyoxyethylene fatty acid ester, polyhydric alcohol ester and the higher fatty acid glycerin ester, an alkanolamide and alkyl polyglucoside. Each of these nonionic surfactants can be used in the composition according to the present invention alone, or two or more kinds of them can be used in combination.

The composition according to the present invention preferably contains a polyoxyethylene type nonionic surfactant and more preferably contains a polyoxyethylene alkyl ether from the view point of emulsified state and storage stability of the composition. The alkyl group of the polyoxyethylene alkyl ether is preferably, for example, cetyl, cetostearyl, stearyl and behenyl from the view point of storage stability of the composition. For example, the use of a polyoxyethylene alkyl ether such as polyoxyethylene(20EO) cetostearyl ether, polyoxyethylene (30EO) cetostearyl ether, polyoxyethylene (40EO) cetostearyl ether, polyoxyethylene (20EO) cetyl ether, polyoxyethylene (30EO) cetyl ether and polyoxyethylene (40EO) cetyl ether is very particularly preferable.

In the composition according to the present invention, the total weight (B) of (b1) and (b2) is from 0.5 to 1.8 % by weight based on the total amount of the composition according to the present invention so as to obtain good storage stability of the composition according to the present invention and good firmness/resilience and good manage ability of hair after a dyeing or bleaching treatment. The total weight (B) of (b1) and (b2) is preferably from 1 to 1.5 % by weight based on the total amount of the composition according to the present invention from the view point of storage stability of the composition according to the present invention and firmness/resilience and manage ability of hair after the treatment.

In the composition according to the present invention, the weight ratio (b1)/(b2) of (b1) to (b2) is preferably from 0.1 to 1 and more preferably from 0.3 to 0.8 from the view point of storage stability of the composition according to the present invention, operability of the dyeing or bleaching agent composition and firmness/resilience and manage ability of hair after a dyeing or bleaching treatment.

The composition according to the present invention contains these components in the amount that the weight ratio (A)/(B) of the total weight (A) to the total weight (B), in which the total weight (A) is the total weight of (a1) oil which is in a liquid form at normal temperature and (a2) oil which is in a solid form at normal temperature and the total weight (B) is a total weight of (b1) the cationic surfactant and (b2) the nonionic surfactant, is from 5 to 30 so as to obtain good emulsified state and good storage stability of the composition and good firmness/resilience and manage ability of hair after a dyeing or bleaching treatment. The weight ratio (A)/(B) of the total weight (A) to the total weight (B) is preferably from 10 to 20 from the view point of emulsified state and storage stability of the composition according to the present invention.

The composition according to the present invention contains the higher alcohol which is in a solid form at normal temperature and (b2) the nonionic surfactant in such an amount that the weight ratio (the higher alcohol)/(b2) of (the higher alcohol) to (b2) is preferably from 1 to 10 and more preferably from 3 to 7.

### (C) Oxidizing agent

The composition according to the present invention contains an oxidizing agent so as to obtain a sufficient hair dyeing or hair bleaching effect. The oxidizing agent is not particularly limited. The examples of the oxidizing agent include, for example, hydrogen peroxide, sodium perborate, potassium perborate, sodium percarbonate and sodium bromate. Each of these oxidizing agents can be used in the composition according to the present invention alone, or two or more kinds of them can be used in combination. Among these oxidizing agents, hydrogen peroxide is preferable since a sufficient dyeing or bleaching power can be obtained.

The amount of the oxidizing agent is from 0.1 to 12 % by weight, preferably from 0.5 to 9 % by weight, even more preferably from 1 to 6 % by weight based on the total weight of the second agent composition from the view point of a sufficient hair dyeing or hair bleaching effect, and irritation to hair or scalp.

The composition according to the present invention has a pH of preferably from 2 to 5 and more preferably from 2 to 4 (determined at 25 °C) from the view point of storage stability of the composition.

The composition according to the present invention may contain a pH adjuster so as to adjust the pH of the second agent composition. The pH adjuster is not particularly limited. For example, an inorganic acid, an organic acid or salts thereof may be used as the pH adjuster. The examples of the inorganic acid include hydrochloric acid and phosphoric acid. The examples of the organic acid include, for example, citric acid, glycol acid, succinic acid, tartaric acid, lactic acid, malic acid, levulinic acid, acetic acid, butyric acid, valeric acid, oxalic acid, maleic acid, fumaric acid and mandelic acid. The examples of the salts thereof include a hydrochloride, a sodium salt, a potassium salt and an ammonium salt thereof. For example, sodium pyrophosphate and phosphoric acid are preferably used.

The composition according to the present invention may contain, in addition to the components described above, components or additives such as a hair care agent, a thickener, a wetting agent, a penetrating agent, an ultraviolet light absorbent, a preservative, a chelating agent, a flavoring agent, a coloring agent and a hair growth stimulating agent which are usually used in cosmetics. The composition according to the present invention may appropriately contain these components and additives in any combination as far as they do not deteriorate the effect of the present invention.

The composition according to the present invention is a second agent composition for hair dyeing or bleaching. A dyeing or bleaching agent composition obtained by mixing the composition according to the present invention with a first agent composition containing an alkali agent and optionally with other composition immediately before use can dye or bleach hair by being applied to hair.

The dosage form of the first agent composition and the second agent composition may be a liquid form, an emulsion form, a cream form, a gel form, a paste form, a foam form and an aerosol form, but the dosage form is not limited to these forms. In addition, the first agent composition and the second agent composition may be different to each other in the dosage form, for example, the first agent may be a cream form and the second agent composition may be a liquid form. In addition, the mixing ratio of the first agent composition and the second agent composition is not limited. The dosage form of the first agent composition and the second agent composition is preferably a cream form from the view point of stability of the composition and operability of the dyeing or bleaching agent composition.

In addition to the first agent composition and the second agent composition, other composition may be mixed immediately before use. The examples of other composition include, for example, a third agent composition containing an oxidization auxiliary agent. The oxidization auxiliary agent contained in the third agent composition promotes decomposition of the oxidizing agent contained in the second agent composition and further enhances decomposition of melanin in hair by generated oxygen when the first agent composition, the second agent composition and the third agent composition are mixed. The dosage form of the third agent composition may be a powder form and an anhydrous cream form, but the dosage form is not limited to these forms. The examples of the oxidization auxiliary agent contained in the third agent composition include, for example, a persulfate such as ammonium persulfate, potassium persulfate and sodium persulfate. Each of these persulfates can be contained in the third agent composition alone, or two or more kinds of them can be contained in combination.

If the first agent composition containing an alkali agent contains dyes, a dyeing agent composition can be obtained by mixing the first agent composition with the second agent composition and optionally with other composition. If the first agent composition containing an alkali agent does not contain any dye, a bleaching agent composition can be obtained by mixing the first agent composition with the second agent composition and optionally with other composition.

The alkali agent is not particularly limited. For example, ammonia, an alkanol amine such as monoethanol amine (MEA), diethanol amine (DEA), triethanol amine (TEA), 2-amino-2-methyl-1-propanol (AMP), 2-amino-2-methyl-1,3-propanediol (AMPD), tetrakis (2-hydroxy isopropyl) ethylene diamine (TE) and monoisopropanol amine (MIPA), ammonium carbonate, ammonium bicarbonate, sodium carbonate, potassium carbonate, sodium phosphate, disodium hydrogen phosphate, sodium hydroxide and potassium hydroxide may be used as the alkali agent. The dyes are not particularly limited. An oxidation dye and a direct dye which are usually used in cosmetic field may be used as the dye.

The pH of the first agent composition is preferably from 8 to 12 and more preferably from 9 to 11 from the view point of dyeing or bleaching power and skin irritation. In addition, the pH of the dyeing or bleaching agent composition obtained by mixing the first agent composition with the second agent composition is preferably from 8 to 12 and more preferably from 8 to 10 from the view point of dyeing or bleaching power and skin irritation.

The composition according to the present invention can be used by being mixed with the first agent composition in a usual process depending on the dosage form as far as the effect of the present invention is not deteriorated. For example, the process may be appropriately selected from the following processes: a process in which the first agent composition is mixed with the second agent composition to be uniform by the use of a brush, a muddler or a spatula in a tray or the like used as a container, then, the resulting composition is applied to hair by the use of a brush, a comb or a hand, and a process in which the first agent composition and the second agent composition are placed in a container having sufficient capacity, then the container is shaken to obtain a uniformly mixed composition, and the resulting composition is applied to hair by the use of an applicator for application (such as a single nozzle and a comb type nozzle), a brush, a comb or a hand.

The composition is preferably applied to hair at a temperature from 15 °C to 45 °C from the view point of burdens on hair and a human body. The time period for bleaching or dyeing after the application of the composition to hair is appropriately regulated depending on the content of the alkali agent or the oxidizing agent, the kind and amount of the oxidation dye, the amount of the composition applied to hair and a desired degree of dyeing. The time period is usually from 3 to 45 minutes, preferably from 5 to 30 minutes and more preferably from 10 to 30 minutes. Then, after passage of the time period for bleaching or dyeing, the hair is washed and optionally dried as necessary.

### [Examples]

Hereinafter, Examples of the present invention are described together with Comparative Examples. The present invention is not limited to the following Examples. In Examples and Comparative Examples, the content is described as % by weight.

The first agent composition having the formulation described in Table 1 was prepared by a conventional method. The second agent compositions having the formulations described in Tables 2 to 4 were prepared by a conventional method.

### (Emulsified State)

Emulsified state of each second agent composition described in Tables 2 to 4 was visually observed and evaluated according to the following levels. The results are shown in Tables 2 to 4.
"Superior" : Emulsified state is very good.
"Good" : Emulsified state is good although the particles are a little coarser than the particles of the emulsified state of "Superior".
"poor" : Emulsified state is inhomogeneous and bad.
"very poor" : The composition is not emulsified and cannot form a cream.

### (Storage Stability)

The second agent compositions were stored in a glass container at 50 °C for one month. Then, the change in appearance was visually observed and evaluated according to the following levels. The results are shown in Tables 2 to 4.
"Superior" : No change
"Good" : Almost no change
"poor" : Degradation in viscosity
"very poor" : Separation

The first agent composition having the formulation described in Table 1 was mixed with the second agent composition having each formulation described in Tables 2-4 in equal amount to obtain a dyeing agent composition. The resulting dyeing agent composition was applied to human head by the use of a blush. At that time, operability was evaluated. After the applied dyeing agent composition was left for 30 minutes, the dyeing agent composition was washed out. Then, the hair was treated with a shampoo and a conditioner and dried by the use of a dryer. Hair after the dyeing treatment was evaluated regarding firmness/resilience, manage ability, feeling, dyeing power and dyeing evenness according to the following levels. The results are shown in Tables 2 to 4.

### (Operability)

"Superior" : Completely no liquid dripping occurred and the composition can be applied to hair very easily.
"Good" : No liquid dripping occurred and the composition can be applied to hair easily.
"poor" : No liquid dripping occurred, but the composition cannot be applied to hair easily.
"very poor" : Liquid dripping occurred and the composition cannot be applied to hair easily.

### (Firmness/Resilience)

"Superior" : The hair has very good resilience and flexibility.
"Good" : The hair has good resilience and flexibility.
"poor" : The hair has not much resilience and flexibility.
"very poor" : The hair has no resilience and flexibility.

### (Manage Ability)

"Superior" : Manage ability of hair is very good.
"Good" : Manage ability of hair is good.
"poor" : Manage ability of hair is bad.
"very poor" : Manage ability of hair is very bad.

### (Feeling)

"Superior" : The feeling of hair is very smooth to the touch.
"Good" : The feeling of hair is smooth to the touch.
"poor" : The hair is difficult to get fingers through and there is a creakiness of hair.
"very poor" : The hair is very difficult to get fingers through and fingers are caught by hair.

### (Dyeing Power)

"Superior" : Hair is dyed very well.
"Good" : Hair is dyed well.
"poor" : Hair is not dyed well.
"very poor" : Hair is scarcely dyed.

### (Dyeing Evenness)

"Superior" : There is no unevenness and hair is very uniformly dyed.
"Good" : There is almost no unevenness and hair is uniformly dyed.
"poor" : There is slight unevenness.
"very poor" : There is much unevenness.

**[Table 1]**

| First Agent Composition | |
|---|---|
| Constituent Name | Compounded Amount |
| Cetostearyl Alcohol | 6 |
| Stearyl Trimethylammonium Chloride (80 %) | 3 |
| Isopropyl Palmitate | 5 |
| Glycerine | 1 |
| POE (30EO) Cetostearyl Ether | 2 |
| Aqueous Ammonia (28 %) | 3 |
| Monoethanol Amine | 3 |
| Ammonium Bicarbonate | 1 |
| Tetrasodium Edetate Tetrahydrate | 0.2 |
| Sodium Sulfite | 0.4 |
| Ascorbic Acid | 0.3 |
| Toluene-2,5-Diamine Sulfate | 0.44 |
| p-Amino Phenol | 0.22 |
| m-Amino Phenol | 0.11 |
| Resorcin | 0.27 |
| 5-Amino o-Cresol | 0.07 |
| Purified Water | q.s. |
| Total | 100 |

**[Table 2]**

| | | | Examples | | | | | | | | | |
|---|---|---|---|---|---|---|---|---|---|---|---|---|
| | | | 1 | 2 | 3 | 4 | 5 | 6 | 7 | 8 | 9 | 10 |
| A | a1 | Isopropyl Palmitate | 20 | 20 | 20 | 20 | | | 20 | 20 | 20 | 25 |
| | | Liquid Paraffin | | | | | 17 | | | | | |
| | | Dioctyl Cyclohexane | | | | | | 17 | | | | |
| | a2 | Cetostearyl Alcohol | 5 | 5 | 5 | 5 | 5 | 5 | | 5 | 5 | 5 |
| | | Cetyl Alcohol | | | | | | | 5 | | | |
| B | b1 | Stearyl Trimethylammonium Chloride (80%) | 0.5 | 0.5 | 0.5 | 0.5 | 0.5 | 0.5 | 0.5 | | | 0.5 |
| | | Cetyl trimethylammonium Chloride (30%) | | | | | | | | 1 | | |
| | | Behenyl Trimethylammonium Chloride (80%) | | | | | | | | | 0.5 | |
| | b2 | POE(20EO) Cetostearyl Ether | 1 | | | | 1 | 1 | 1 | 1 | 1 | 1 |
| | | POE(30EO) Cetostearyl Ether | | 1 | | | | | | | | |
| | | POE(40EO) Cetyl Ether | | | 1 | | | | | | | |
| | | Lauryl Glucoside (50%) | | | | 2 | | | | | | |
| c | | Aqueous Hydrogen Peroxide (35%) | 16.5 | 16.5 | 16.5 | 16.5 | 16.5 | 16.5 | 16.5 | 16.5 | 16.5 | 16.5 |
| Hydroxy Ethane Diphosphonic Acid Liquid (60%) | | | 0.25 | 0.25 | 0.25 | 0.25 | 0.25 | 0.25 | 0.25 | 0.25 | 0.25 | 0.25 |
| Phenoxy ethanol | | | 0.25 | 0.25 | 0.25 | 0.25 | 0.25 | 0.25 | 0.25 | 0.25 | 0.25 | 0.25 |
| Sodium Pyrophosphate | | | Amount for Adjusting pH to 3 | | | | | | | | | |
| Purified Water | | | to 100 | | | | | | | | | |
| (A)/(a2) | | | 5 | 5 | 5 | 5 | 4.4 | 4.4 | 5 | 5 | 5 | 6 |
| (B) | | | 1.4 | 1.4 | 1.4 | 1.4 | 1.4 | 1.4 | 1.4 | 1.3 | 1.4 | 1.4 |
| (b1)/(b2) | | | 0.4 | 0.4 | 0.4 | 0.4 | 0.4 | 0.4 | 0.4 | 0.3 | 0.4 | 0.4 |
| (A)/(B) | | | 17.9 | 17.9 | 17.9 | 17.9 | 15.7 | 15.7 | 17.9 | 19.2 | 17.9 | 21.4 |
| Evaluation Results | | Emulsified State | ⊚ | ⊚ | ○ | ○ | ○ | ○ | ⊚ | ⊚ | ○ | ○ |
| | | Storage Stability | ⊚ | ⊚ | ⊚ | ○ | ⊚ | ⊚ | ⊚ | ⊚ | ○ | ⊚ |
| | | Operability | ⊚ | ⊚ | ○ | ○ | ⊚ | ○ | ⊚ | ⊚ | ○ | ○ |
| | | Firmness/Resilience | ⊚ | ⊚ | ⊚ | ⊚ | ⊚ | ⊚ | ⊚ | ⊚ | ⊚ | ⊚ |
| | | Manage Ability | ⊚ | ⊚ | ⊚ | ⊚ | ⊚ | ○ | ⊚ | ⊚ | ⊚ | ⊚ |
| | | Feeling | ⊚ | ⊚ | ⊚ | ⊚ | ⊚ | ○ | ⊚ | ⊚ | ⊚ | ○ |
| | | Dyeing Power | ⊚ | ⊚ | ⊚ | ⊚ | ⊚ | ⊚ | ⊚ | ⊚ | ⊚ | ⊚ |
| | | Dyeing Evenness | ⊚ | ⊚ | ⊚ | ⊚ | ⊚ | ⊚ | ⊚ | ⊚ | ⊚ | ⊚ |

| | | | | | | | | | | | | |
|---|---|---|---|---|---|---|---|---|---|---|---|---|
| ⊚: Superior, ○: Good, Δ: Poor, ×: Very Poor | | | | | | | | | | | | |

**[Table 3]**

| | | | Examples | | | | | | Comparative Examples | | |
|---|---|---|---|---|---|---|---|---|---|---|---|
| | | | 11 | 12 | 13 | 14 | 15 | 16 | 1 | 2 | 3 |
| A | a1 | Isopropyl Palmitate | 30 | 20 | 20 | 20 | 10 | 10 | 20 | | 20 |
| | | Liquid Paraffin | | | | | | | | 17 | |
| | | Dioctyl Cyclohexane | | | | | | | | | |
| | a2 | Cetostearyl Alcohol | 5 | 4 | 7 | 5 | 5 | 5 | 5 | 5 | 5 |
| | | Cetyl Alcohol | | | | | | | | | |
| B | b1 | Stearyl Trimethylammonium Chloride (80%) | 0.5 | 0.5 | 0.5 | 1 | 0.5 | 0.3 | 0.5 | 0.5 | 3 |
| | | Cetyl trimethylammonium Chloride (30%) | | | | | | | | | |
| | | Behenyl Trimethylammonium Chloride (80%) | | | | | | | | | |
| | b2 | POE(20EO) Cetostearyl Ether | 1 | 1 | 1 | 1 | 0.6 | 0.3 | 6 | 6 | 1 |
| | | POE(30EO) Cetostearyl Ether | | | | | | | | | |
| | | POE(40EO) Cetyl Ether | | | | | | | | | |
| | | Lauryl Glucoside (50%) | | | | | | | | | |
| c | | Aqueous Hydrogen Peroxide (35%) | 16.5 | 16.5 | 16.5 | 16.5 | 16.5 | 16.5 | 16.5 | 16.5 | 16.5 |
| Hydroxy Ethane Diphosphonic Acid Liquid (60%) | | | 0.25 | 0.25 | 0.25 | 0.25 | 0.25 | 0.25 | 0.25 | 0.25 | 0.25 |
| Phenoxy ethanol | | | 0.25 | 0.25 | 0.25 | 0.25 | 0.25 | 0.25 | 0.25 | 0.25 | 0.25 |
| Sodium Pyrophosphate | | | Amount for Adjusting pH to 3 | | | | | | | | |
| Purified Water | | | to 100 | | | | | | | | |
| (A)/(a2) | | | 7 | 6 | 3.86 | 5 | 3 | 3 | 5 | 4.4 | 5 |
| (B) | | | 1.4 | 1.4 | 1.4 | 1.8 | 1 | 0.54 | 6.4 | 6.4 | 3.4 |
| (b1)/(b2) | | | 0.4 | 0.4 | 0.4 | 0.8 | 0.67 | 0.8 | 0.07 | 0.07 | 2.4 |
| (A)/(B) | | | 25 | 17.1 | 19.3 | 13.9 | 15 | 27.8 | 3.91 | 3.44 | 7.35 |
| Evaluation Results | | Emulsified State | ○ | ○ | ○ | ○ | ○ | ○ | Δ | Δ | ○ |
| | | Storage Stability | ○ | ⊚ | ⊚ | ⊚ | ⊚ | ○ | Δ | Δ | Δ |
| | | Operability | ○ | ○ | ○ | ○ | ⊚ | ⊚ | Δ | Δ | Δ |
| | | Firmness/Resilience | ⊚ | ⊚ | ⊚ | ○ | ○ | ○ | ○ | ○ | Δ |
| | | Manage Ability | ⊚ | ○ | ○ | ○ | ○ | ○ | ○ | ○ | Δ |
| | | Feeling | ⊚ | ⊚ | ⊚ | ⊚ | ○ | ○ | Δ | Δ | ○ |
| | | Dyeing Power | ⊚ | ⊚ | ○ | ⊚ | ○ | ○ | ○ | ○ | ○ |
| | | Dyeing Evenness | ⊚ | ○ | ○ | ⊚ | ⊚ | ⊚ | ○ | Δ | Δ |

| | | | | | | | | | | | |
|---|---|---|---|---|---|---|---|---|---|---|---|
| ⊚: Superior, ○: Good, Δ: Poor, ×: Very Poor | | | | | | | | | | | |

**[Table 4]**

| | | | Comparative Examples | | | | | | | |
|---|---|---|---|---|---|---|---|---|---|---|
| | | | 4 | 5 | 6 | 7 | 8 | 9 | 10 | 11 |
| A | a1 | Isopropyl Palmitate | 20 | | | 20 | 20 | 20 | 20 | 5 |
| | | Liquid Paraffin | | 20 | 20 | | | | | |
| | | Dioctyl Cyclohexane | | | | | | | | |
| | | Amodimethicone | | | | | | | | 0.2 |
| | a2 | Cetostearyl Alcohol | 2 | 7 | | 5 | 5 | 5 | 5 | 5 |
| | | Cetyl Alcohol | | | 7 | | | | | |
| B | b1 | Stearyl Trimethylammonium Chloride (80%) | 0.5 | 4 | 3 | | | | | 1 |
| | | Cetyl Trimethylammonium Chloride (30%) | | | | | | | | |
| | | Behenyl Trimethylammonium Chloride (80%) | | | | | | | | |
| | | Distearyl Dimethylammonium Chloride (80%) | | | | | | 0.5 | | |
| | | Tri(polyoxyethylene) Stearylammonium Chloride (5.E.O)(20%) | | | | | | | 2 | |
| | b2 | POE(20EO) Cetostearyl Ether | 1 | 4 | 3 | 1 | 1.4 | 1 | 1 | 3 |
| | | POE(30EO) Cetostearyl Ether | | | | | | | | |
| | | POE(40EO) Cetyl Ether | | | | | | | | |
| | | Lauryl Glucoside (50%) | | | | | | | | |
| c | | Aqueous Hydrogen Peroxide (35%) | 16.5 | 16.5 | 16.5 | 16.5 | 16.5 | 16.5 | 16.5 | 16.5 |
| Dimethyl Diallyl ammonium Chloride Copolymer | | | | | | | | | | 0.1 |
| Glycerin | | | | 5 | 5 | | | | | |
| Hydroxy Ethane Diphosphonic Acid Liquid (60%) | | | 0.25 | 0.25 | 0.25 | 0.25 | 0.25 | 0.25 | 0.25 | 0.25 |
| Phenoxy ethanol | | | 0.25 | 0.25 | 0.25 | 0.25 | 0.25 | 0.25 | 0.25 | 0.25 |
| Sodium Pyrophosphate | | | Amount for Adjusting pH to 3 | | | | | | | |
| Purified Water | | | to 100 | | | | | | | |
| (A)/(a2) | | | 11 | 3.86 | 3.86 | 5 | 5 | 5 | 5 | 2 |
| (B) | | | 1.4 | 7.2 | 5.4 | 1 | 1.4 | 1.4 | 1.4 | 3.8 |
| (b1)/(b2) | | | 0.4 | 0.8 | 0.8 | 0 | 0 | 0.4 | 0.4 | 0.27 |
| (A)/(B) | | | 15.7 | 3.8 | 5.0 | 25.0 | 17.9 | 17.9 | 17.9 | 2.63 |
| Evaluation Results | | Emulsified State | Δ | Δ | ○ | Δ | Δ | Δ | Δ | ○ |
| | | Storage Stability | × | Δ | Δ | × | × | × | × | Δ |
| | | Operability | Δ | Δ | Δ | Δ | Δ | Δ | Δ | Δ |
| | | Firmness/Resilience | ○ | Δ | Δ | Δ | Δ | Δ | Δ | Δ |
| | | Manage Ability | ○ | Δ | Δ | Δ | Δ | Δ | Δ | Δ |
| | | Feeling | Δ | ○ | ○ | Δ | Δ | ○ | ○ | ○ |
| | | Dyeing Power | ○ | ○ | ○ | ○ | ○ | ○ | ○ | ○ |
| | | Dyeing Evenness | ○ | Δ | Δ | Δ | Δ | Δ | Δ | ○ |

| | | | | | | | | | | |
|---|---|---|---|---|---|---|---|---|---|---|
| ⊚: Superior, ○: Good, Δ: Poor, ×: Very Poor | | | | | | | | | | |

## Claims

1. A second agent composition for hair dyeing or bleaching which is used by being mixed with a first agent composition containing an alkali agent, wherein the second agent contains following components (A) to (C):
(A) (a1) from 10 to 30 % by weight of at least one oil which is in a liquid form at 25°C and (a2) at least one oil which is in a solid form at 25°C, wherein, the oil which is in a solid form at 25°C comprises a higher alcohol which is in a solid form at 25°C, and the weight ratio (A)/(a2) of the total weight (A) of (a1) and (a2) to (a2) is from 3 to 8,
(B) (b1) at least one cationic surfactant and (b2) at least one nonionic surfactant, wherein the cationic surfactant comprises an alkyltrimethylammonium chloride which has from 16 to 22 carbon atoms in the alkyl group thereof, and the total weight (B) of (b1) and (b2) is from 0.5 to 1.8 % by weight,
and,
(C) from 0.1 to 12 % by weight of an oxidizing agent, wherein, the weight ratio (A)/(B) of (A) to (B) is from 5 to 30.

2. The composition according to claim 1, wherein the weight ratio (b1)/(b2) of (b1) to (b2) is from 0.1 to 1.

3. The composition according to claim 1 or 2, wherein the higher alcohol comprises at least one selected from the group consisting of cetyl alcohol, stearyl alcohol and cetostearyl alcohol.

4. The composition according to any one of claims 1 to 3, wherein the nonionic surfactant comprises at least one polyoxyethylene alkyl ether.

## Patentansprüche

1. Zusammensetzung eines zweiten Mittels zum Haarfärben oder -bleichen, die durch Mischen mit einer ein Alkalimittel enthaltenden Zusammensetzung eines ersten Mittels verwendet wird, wobei das zweite Mittel folgende Komponenten (A) bis (C) enthält:
(A) (a1) 10 bis 30 Gew.-% mindestens eines Öls, das bei 25 °C in einer flüssigen Form vorliegt, und (a2) mindestens eines Öls, das bei 25 °C in einer festen Form vorliegt, wobei das Öl, das bei 25 °C in einer festen Form vorliegt, einen höheren Alkohol umfasst, der bei 25 °C in einer festen Form vorliegt, und das Gewichtsverhältnis (A)/(a2) des Gesamtgewichts (A) von (a1) und (a2) zu (a2) von 3 bis 8 beträgt,
(B) (b1) mindestens ein kationisches Tensid und (b2) mindestens ein nichtionisches Tensid, wobei das kationische Tensid ein Alkyltrimethylammoniumchlorid umfasst, das 16 bis 22 Kohlenstoffatome in der Alkylgruppe davon aufweist und das Gesamtgewicht (B) von (b1) und (b2) 0,5 bis 1,8 Gew.-% beträgt,
und
(C) 0,1 bis 12 Gew.-% eines Oxidationsmittels, wobei das Gewichtsverhältnis (A)/(B) von (A) zu (B) 5 bis 30 beträgt.

2. Zusammensetzung nach Anspruch 1, wobei das Gewichtsverhältnis (b1)/(b2) von (b1) zu (b2) 0,1 bis 1 beträgt.

3. Zusammensetzung nach Anspruch 1 oder 2, wobei der höhere Alkohol mindestens einen, ausgewählt aus der Gruppe bestehend aus Cetylalkohol, Stearylalkohol und Cetostearylalkohol, umfasst.

4. Zusammensetzung nach einem der Ansprüche 1 bis 3, wobei das nichtionische Tensid mindestens einen Polyoxyethylenalkylether umfasst.

## Revendications

1. Seconde composition pour teindre ou décolorer les cheveux, qui s'utilise en mélange avec une première composition contenant un agent alcalin, la seconde composition contenant les composants (A) à (C) suivants :
(A)
(a1) 10 à 30 % en poids d'au moins une huile qui est sous forme liquide à 25°C et
(a2) au moins une huile qui est sous forme solide à 25°C, l'huile qui est sous forme solide à 25°C contenant un alcool supérieur qui est sous forme solide à 25°C, et le rapport pondéral (A)/(a2) du poids total (A) de (a1) et (a2) sur (a2) étant de 3 à 8,
(B)
(b1) au moins un tensioactif cationique, et
(b2) au moins un tensioactif non-ionique, le tensioactif cationique contenant au chlorure d'alkyltriméthylammonium ayant de 16 à 22 atomes de carbone dans son groupe alkyle, et
le poids total (B) de (b1) et (b2) étant de 0,5 à 1,8 % en poids, et
(C) 0,1 à 12 % en poids d'un oxydant,
où le rapport pondéral (A)/(B) de (A) à (B) est de 5 à 30.

2. Composition selon la revendication 1, dans laquelle le rapport pondéral (b1)/(b2) de (b1) à (b2) est de 0,1 à 1.

3. Composition selon la revendication 1 ou 2, dans laquelle l'alcool supérieur contient au moins un membre du groupe constitué de l'alcool cétylique, l'alcool stéarique et l'alcool cétostéarylique.

4. Composition selon l'une quelconque des revendications 1 à 3, dans laquelle le tensioactif non-ionique contient au moins un polyoxyéthylène alkyl éther.
